# EUROPEAN PATENT SPECIFICATION

(11) **EP 1 396 264 B1**
(45) Date of publication and mention of the grant of the patent: **10.10.2007**
(21) Application number: 02726472.0
(22) Date of filing: 23.05.2002
(51) Int. Cl.: A61K 31/122, A61K 31/215, A23L 1/30, A61P 27/02, A23L 1/275

(54) **RELIEVING EYE CONTROLLING FUNCTION ERROR**
LINDERUNG VON FEHLERN IN DER AUGENKONTROLLFUNKTION
ATTENUATION D'UNE ERREUR DE FONCTION DE CONTROLE DE L'OEIL

(30) Priority: 24.05.2001 JP 2001155575
(43) Date of publication of application: 10.03.2004
(73) Proprietor: FUJI CHEMICAL INDUSTRY CO., LTD., Nakaniikawa-gun, Toyama 930-0397 (JP)
(72) Inventor: HAYASAKA, Seiji, Toyama-shi, Toyama 939-8206 (JP); NAGAKI, Yasunori, Toyama-shi, Toyama 930-0036 (JP); UONOMI, Takatoshi, Nakaniikawa-gun, Toyama 930-0397 (JP); SANADA, Mari, Nakaniikawa-gun, Toyama 930-0397 (JP)
(74) Representative: Albrecht, Thomas
(86) International application number: PCT/JP2002/005011
(87) International publication number: WO 2002/094253

(56) References cited:
- DE-A1- 19 950 327
- JP-A- 10 276 721
- JP-A- 2002 017 295
- US-A- 5 527 533
- US-B1- 6 200 601

## Description

### Technical Field

The present invention relates to a medicament for improving failure of accommodation comprising astaxanthin and/or its esters, and to food and drink causing an improving effect against failure of accommodation comprising astaxanthin and/or its esters.

### Background Art

The human eyes have an accommodation to automatically accommodate so as to focus always on the retina by thickening the lens for near point vision or otherwise by thinning them for far point vision. The failure of this accommodation include presbyopia which shows the difficulty in near point accommodation caused by poor accommodability due to aged-deterioration, and morbid abnormalities of accommodation such as weakness of accommodation, hypocyclosis, dullness of accommodation, accommodation paralysis, tonic accommodation, accommodation spasm etc. More specifically, causes of the latter disorders may include eye-fatigues such as ciliary fatigue, fatigue of ocular muscle which moves eyeball and fatigue of optic nerves, and systemic diseases or other ophthalmic diseases. In the treatment of these disorders, it is said that no therapeutic method has been existed for the presbyopia which may only be corrected symptomatically with glasses or contact lens in order to improve poor accommodability. For the morbid abnormalities of accommodation, therapy of the underlying disease or the environmental improvement may be performed. Their symptomatic treatment may include correction with glasses or dosing of vitamin B.

Consequently, it is actual situation that there is very few therapeutic methods for failure of the accommodation, especially there is almost no preventive method for them. German patent application DE 199 50 327 A1 relates to the use of esters of carotinoids such as astaxanthin for the prevention and treatment of diseases of the eye and, in particular, in the prevention or treatment of age-related macular degeneration (ARMD) and cataract. A method of using astaxanthin and/or its esters for therapy of retinal damages or retinal diseases is reported (U.S. Patent No. 5,527,533 specification), and it is reported that food and drink comprising astaxanthin and/or its esters permissible as a edible use which have preventive effect against cataract or inhibiting effect against its progression can inhibit crisis or progression of cataract, and furthermore, can inhibit monocular diplopia, asthenopia or halation complicated with disorder of visual acuity associated with cataract (JP 10-276721 A)). However, there is no report on medicaments for improvement of failure of accommodation comprising astaxanthin and/or its esters, or on food and drink having an improving effect against failure of accommodation comprising astaxanthin and/or its esters.

### Disclosure of the Invention

An object of the present invention is to provide a useful medicament for therapy and/or prevention of failure of accommodation and further to provide food and drink having an improving effect against failure of accommodation.

As a result of having searched effective compounds for improvement of failure of accommodation, the present inventors have found that astaxanthin and/or its esters are useful in the preparation of a medicament for improvement of failure of accommodation, and also found that of food and drink containing astaxanthin and/or its esters as component thereof show an improving effect against failure of the accommodation. Thus, the invention has made based on the above findings.

That is, the invention is the use of astaxanthin and/or its esters for the preparation of a medicament for improvement of failure of accommodation, and of food and drink having an improving effect against failure of the accommodation.

Astaxanthin and/or its esters for use as the effective ingredients in the present invention may be chemically synthesized ones, extracts or crude extracts derived from natural origin. Those may be used singly or in the form suitably mixed. Examples of one derived from natural origin include crusts, eggs and organs of crustaceans such as shrimp, krill, crab and the like; skins and eggs of various fishes and shellfishes; algae such as Haematococcus, etc.; yeasts such as *Phaffia* red yeast, etc.; oceanic bacteria such as *Agrobacterium auranticum*; and seed plants such as *Adonis amurensis* and *Ranunculus acris*. Naturally extracted products and synthesized products are put on the marketplace and hence they are easily available.

Astaxanthin and/or its esters can be obtained by cultivation of e.g. *Phaffia* red yeast, Hematococcus green algae, *Agrobacterium auranticum*, etc. in an appropriate medium in accordance with the conventional methods or the known methods.

Various methods are known for extraction of astaxanthin from the above cultivated substances or for extraction and purification from the above crustaceans. For example, since diester form of astaxanthin has liposoluble property, astaxanthin components can be extracted from the natural origin containing astaxanthin with liposoluble organic solvents such as acetone, alcohol, ethyl acetate, benzene, chloroform, etc.. After the extraction, the concentrated diester form of astaxanthin can be obtained by removing the solvents according to a usual method. The concentrated astaxanthin diester can be further purified, if necessary.

Astaxanthin comprises 3,3'-dihydroxy-*β*, *β*-carotene-4,4'-dione and its stereoisomers. More specifically, such three stereoisomers are known as (3R, 3'R)-astaxanthin, (3R, 3'S)-astaxanthin and (3S, 3'S)-astaxanthin. Any of them can be used in the present invention.

It is known that astaxanthin and/or its esters have not been observed having any mutagenicity but are highly safe compounds.

As the astaxanthin component in the present invention, any of free form, monoester and diester forms may be used.
The diester form is more stable physically than the free or monoester form and hard to be subjected to oxidative decomposition, because its two hydroxy groups are protected by ester bondage. However, when it is taken into the living body, it is considered quickly hydrolyzed into free astaxanthin by bioenzymes to exert its effect.

Monoesters of astaxanthin include lower or higher saturated fatty acid esters, or lower or higher unsaturated fatty acid esters. Specifically, the monoesters include such ester forms of acetic acid, lauric acid, myristic acid, pentadecanoic acid, palmitic acid, palmitoleic acid, heptadecanoic acid, elaidic acid, ricinoleic acid, petroselinic acid, vaccenic acid, eleostearic acid, punicinic acid, licanoic acid, palynalic acid, gadolic acid, 5-eicosenoic acid, 5-docosenoic acid, cetolic acid, ercinoic acid, 5,13-docosadienoic acid, selacholic acid, decenoic acid, stering acid, dodecenoic acid, oleic acid, stearic acid, eicosapentaenoic acid, docosahexaenoic acid, linoleic acid, linolenic acid, arachidonic acid, etc

Diesters of astaxanthin include such diesters composed of the same or different fatty acids selected from the above fatty acids.

Furthermore, examples of astaxanthin esters include esters of an amino acid such as glycine, alanine or the like; esters of a mono- or poly-carboxylic acid and their salts such as citric acid esters, etc.; or inorganic acid esters and their salts such as phosphoric acid esters, sulfuric acid esters, etc.; glyco-esters such as glucoside, etc.; mono esters such as glyco-fatty acid esters, glycoglycero-fatty acid esters, sphingoglyco-fatty acid esters, glycero-fatty acid esters, glycero-phosphoric acid esters, etc. Or they include the same or different diesters selected from the above amino acids, carboxylic acids, phosphoric acids, sulfuric acids, sugars, unsaturated fatty acids, saturated fatty acids, polyunsaturated fatty acids, fatty acid esters, glyco-fatty acid esters, glycoglycero-fatty acid esters, shpingoglyco-fatty acid esters, glycero-fatty acid esters, glycero-phosphoric acid esters, etc.

The medicament obtained by the use of the present invention which is the medicament for improving failure of the accommodation comprising astaxanthin and/or its esters can be prepared in various dosing forms according to the conventional methods, by appropriately combining with sugars such as lactose, saccharose, etc., amino acids such as glycine, etc., excipients such as cellulose, etc., binders such as starch, gelatin, methyl cellulose, polyvinylpyrrolidone, etc., disintegrators such as starch, agar, etc., or lubricants such as silicon dioxide, talc, magnesium stearate, polyethylene glycol, etc., flavors and sweetening agents. For example, the medicament will be administered in such dosing forms as, solid forms such as tablets, powders, granules, fine granules, pills, enteric coated forms, capsules, troche, etc., oral liquid preparations such as elixirs, syrups, etc., liquid forms such as suspensions, emulsions, syrups, external liquid preparations, fomentations, nasal drops, ear drops, eye drops, etc., or capsules filling oil or fat such as soft capsules, etc., inhalants, lotions, suppositories, enteral nutrients, etc.

Astaxanthin and/or its esters are easily oxidized by aerial oxygen and unstable against temperature or light. They show a tendency to be decomposed with the passage of time during storage in their preparation. In order to avoid such decomposition, antioxidants, as stabilizers, can be added to the above components, if necessary. For example, one or two or more mixtures selected from such existing antioxidants as, e.g. vitamin A, vitamin B, vitamin C and vitamin E (tocopherol and tocotrienol) or their derivatives, cysteine, glutathione, phytic acid, catechins, flavonoids, *β*-carotene, glutathione peroxidase, citric acids, phosphoric acids, polyphenols, nucleic acids, herb medicines, marine algae, inorganic substances can be added to the above components. It is desirable to dose them in a fine powder form or non-crystalline powder form in order to increase absorbability of free or monoester astaxanthin.

Although dosage of astaxanthin and/or its esters to be used as the medicament may vary in age, body weight or grade of symptoms of a patient who receives the medicament, or its dosing form, the dose in terms of free astaxanthin will be ranged for adult in oral administration per day : 0.1 mg - 10 g, preferably 0.1 mg - 1 g and preventively 0.1 mg - 100 mg, and in parenteral administration per day : 0.01 mg - 1 g, preferably 0.01 mg - 100 mg and preventively 0.01 mg - 10 mg.

Although the means to be administered is not limited particularly, it is recommendable to administer astaxanthin and/or its esters preferably during hunger or 30 minutes before meal for good efficiency.

As the medicament obtained by the use of the present invention increases the human eye-accommodability, it is useful as a preventive and/or therapeutic agent for such condition of causing failure of the eye-accommodability as, i.e. presbyopia difficult in near point accommodation caused by poor accommodability due to aged-deterioration, weary eyes of a person who works for VDT operation, or patients with such morbid abnormalities as weakness of accommodation, hypocyclosis, dullness of accommodation, accommodation paralysis, tonic accommodation, accommodation spasm, etc.

Incidentally, even though the content disclosed in US Patent No. 5,527,533 specification is directed to "eye", it is restricted to retina and its linked nerve. Also, JP 10-276721 A discloses merely with respect to cataract and its causing asthenopia. On the other hand, the medicament for improving failure of accommodation involving in the present invention is considered that morbid abnormalities of accommodation may be ameliorated by amelioration in bloodstream to the ciliary body and preventing injuries to the ciliary body muscle and by control nerve (parasympathetic).

The present invention relates also to the preparation of a food and a drink having an improving effect against failure of accommodation which comprises astaxanthin and/ or its esters.

The food and the drink to which astaxanthin and/or its esters are added include such general foods as, e.g. margarine, butter, butter sauce, cheese, raw cream, shortening, lard, ice cream, yogurt, diary products, meat sauce products, fish products, fried potato, potato chips, popcorn, a seasoned powder for spinkling over rice, chewing gum, chocolate, pudding, jelly, gumi-candy, candy, drops, caramel, sponge cake, cake, doughnut, biscuit, cookie, cracker, etc., macaroni, pasta, salad oils, instant soup, dressing, egg, mayonnaise, miso., etc., or carbonated or non-carbonated drinks such as fruit drinks, refreshing drinks, sports drinks, etc., non-alcoholic drinks such as tea, coffee, cocoa, etc., or liquors such as liqueur, medical liquor, etc.

The food and the drink obtained by the use of the present invention can be processed by usual methods, combining astaxanthin and/or its esters with raw materials of the general foods. Although the combining quantity of astaxanthin and/or its esters may be varied depending on the food form and so on, generally, it is desirable that the combining quantity as free astaxanthin lies in a range of 0.1 mg - 10 g, preferably 1 mg - 1 g and preventively 0.1 mg - 100 mg. For foods and drinks, functional foods and nutritional supplements, the combining quantity will be adjusted in preparations with necessary quantity to exert the improving effect against failure of the accommodation. The quantity for usage can be selected appropriately depending on the kind of food and drink by persons having ordinary skill in the art.

When the food and drink obtained by the use of the present invention are used as nutritional and supplemental foods or functional foods, their forms may be the same as the above-described medicament forms. There may also be used mixture of such material as milk protein, soybean protein, egg albumin protein, etc., or their decomposed material such as albumen oligopeptide, soybean hydrolyzate, amino acid unit. The food can also be formed into natural liquid foods, semi-digested nutritional foods and nutritional foods, drinks, capsules or enteral nutrients, etc. combining with sugars, fats, trace elements, vitamins, emulsions, flavors, etc. For the drink form, such material can be combined with the drink as nutritional additives such as amino acids, vitamins, minerals, etc., and sweetening agents, spices, flavors, pigments, etc., in order to keep a balance in the components or to impart good taste for taking. Furthermore, such natural extracts as blueberry extract, etc. containing a large amount of anthocyanin which may be good for eye may be added, thereby the synergic effect may be exerted. The form of the food, etc. in the present invention is not limited thereto.

### The Best Mode For Carrying Out The Invention

The following Examples and Preparation Examples illustrate the present invention in details.

### Example 1: Improving effect of astaxanthin on eye-accommodability

### (Test method) A person satisfying the following selection standards was indicated as the subject.

(1) A person having subjective symptoms of eyestrain or working for VDT operation, (2) 1.0 or more of both eyes in vision after correction. (3) 35-59 years old, (4) persons who do not have usual-take of any medicines or health foods, (5) a person who can keep compliance with all the test-related requirements and can take medical examinations stipulated by the test method

Persons who have retinal disorders or cataract were excluded from the subjects.

A test food with 5 mg /capsule of astaxanthin and a control food with 0 mg /capsule of astaxanthin were prepared. The test was conducted in a double-blind method.

### I. Before intake

After completion of the accommodability test for the subjects using an accommodometer by which the change in the refraction value (accommodation reaction) during moving object can be measured continuously and objectively, and accommodation abnormalities including VDT syndrome can be detected clearly.

A person in charge of the test prepared a subjects' name-list stratified by sex and the test results and handed it to a controller. The controller prepared an allocation table separating the subjects into test food group and control food group based on the name list. In addition, the controller stuck each label with each subject's name on the test food or control food according to the allocation table. The controller sealed up the allocation table.

### II. During intake

Each of the subjects took one capsule a day after supper continuously for 4 weeks.

### III. After completion of intake

Each of the subjects took the accommodation examination by the accommodometer. The results are shown in table 1 which indicates the human eye-accommodabilites in the test food group and the control group.

Incidentally, the value of accommodability (dioptres) in table 1 is represented in mean ± standard deviation and " * " in table 1 means significant difference p<0.01, before intake vs. after intake (t-test).

**Table 1**

| | | Number | Accommodability (Dioptres) |
|---|---|---|---|
| Test food Group | Before Intake | 26 | 2.279±1.442 |
| | After Intake | 26 | 2.775±1.563* |
| Control food Group | Before Intake | 30 | 2.551±1.744 |
| | After Intake | 30 | 2.728±1.974 |

It was recognized from the results shown in table 1 that when the human's eye-accommodability was compared between before intake of astaxanthin and after intake of astaxanthin for 4 consecutive weeks, it was increased in the test food group with statistically significant difference. Contrary thereto, such difference was not recognized in the control group. It can be understood that astaxanthin improves the eye-accommodability.

### Example 2

Effects of astaxanthin on eye-accommodability, critical flicker fusion (CFF) and pattern visual evoked potentials (PVEP) were evaluated in more details.

As control, 13 persons who have not took the administration of astaxanthin and have not worked for VDT operation were indicated as A group. 26 VDT workers were divided into two groups at random. B group (13 persons) was orally administered with 5 mg of astaxanthin/day for 4 consecutive weeks while C group (13 persons) was orally administered with placebo for 4 consecutive weeks. No significant difference was recognized in age among the three groups.

A double-blind test was conducted for B and C groups.

The eye-accommodability of A group was 3.7±1.5 dioptres. Each eye-accommodability of B and C groups before administration was 2.3±1.4 dioptres and 2.2±1.0 dioptres, respectively and significantly (p<0.05) lower than that of A group.

The eye-accommodability of 2.8±1.6 dioptres in B group after administration of astaxanthin became significantly (p< 0.01) greater than that before administration of astaxanthin. On the other hand, the eye-accommodability (2.3±1.1 dioptres) in C group after administration of placebo did not (appreciably) change.

With respect to the eye-accommodability, the following values of normal persons by ages are known:

8 Years of age -13.8 dioptres, 16 years of age - 12.0 dioptres, 24 years of age - 10.2 dioptres, 32 years of age - 8.2 dioptres, 40 years of age - 5.8 dioptres, 48 years of age - 2.5 dioptres, 56 years of age - 1.25 dioptres, 64 years of age - 1.1 dioptres ("Stedman's Medical Dictionary" the fourth edition, p. 615)

The critical flicker fusion, the amplitude and the latency of P100 in the pattern visual evoked potentials in A group were 45 ±4.2 Hz, 6.5±1.8 *µ*V. 101.3±6.5 msec, respectively.

The critical flicker fusion was significantly (p<0.01) lower in B and C groups before administration than in A group.

The critical flicker fusion in B and C groups did not (appreciably) change after administration. The amplitude and the latency of P100 in the pattern visual evoked potentials in B and C groups before administration were same with those in A group. They did not appreciably change after administration.

It is suggested from the findings of this study that the eye-accommodability of the VDT workers may be improved after administration of astaxanthin.

VDT operation is reported to induce various visual disorders including eyestrain, blurring and double vision (such a status that single object is observed as two objects) and to have adverse effect on the visual system.

The eye-accommodability, the amplitude and the prolonged latency in the pattern visual evoked potentials are used for determining the degree of eyestrain.

### Subjects and method:

13 persons who had not been engaged in VDT operation were indicated as healthy control group (A group). Most of them work outdoors.

Also, 26 workers were selected who had been engaged in VDT operation for 4 hours per day, for 5 days (Monday to Friday every week) per week and for a year or more. Their eyes were better than twenty-twenty (20/20). All of them wore eyeglasses for accommodation during VDT operation.

Herein, persons who have worn contact lens, ones who have used eye drops within the past 6 months, ones who have suffered from heavy ocular including diabetes mellitus and ones who have suffered from systemic disease were excluded from the subjects.

A double-blind test was conducted with respect to VDT workers. The VDT workers were divided into astaxanthin-administration group (n=13, B group) and placebo-administration group (n=13, C group). There was no difference in age among the three groups (table 2 shown below).

**Table 2**

| | Non-VDT workers | VDT workers | |
|---|---|---|---|
| | A group | B group | C group |
| Number of Subject | 13 | 13 | 13 |
| Male | 11 | 11 | 10 |
| Female | 2 | 2 | 3 |
| Average of age | 47.6±4.5 | 47.8±4.3 | 47.5±4.8 |
| Range of age | 39-53 | 40-53 | 38-53 |

Astaxanthin capsule (5 mg/capsule) was orally administrated to each of B group subjects one time a day 30 minutes before supper.

Astaxanthin was prepared from *Haematococcus pluvialis* extract (a product of Fuji Chemical Industry Co., Ltd.).

Placebo capsule was orally administrated to each of C group subjects one time a day 30 minutes before supper.

B and C group subjects did a usual VDT operation in the administration period. A group subjects did not receive any administration.

### Measurement for the eye-accommodability, the critical flicker fusion and the pattern visual evoked potentials:

All of these measurement items were conducted with each right eye of the subjects at a.m. 9:00-12:00 on Saturday.

Eyesight was measured at each distance of 5 m and 35 cm using Landolt ring.

The eye-accommodability was evaluated by measurement of the near and far points.

The near point was measured with D'Acomo apparatus (binocular opening constant point refraction near point ruler, a product of World Optical Corporation) according to the Uozato et al' method (Uozato H, Nagakawa A, Hirai H, Saishin M : A new near-point ruler using constant dioptric stimulus. Folia Ophthalmol Jpn 1988 : 39 : 1247-1248)).

The far point was measured in the best-corrected refraction for each of the subjects.

The eye-accommodability (dioptres) was calculated by subtracting the far point (dioptres) from the near point (dioptres).

The critical flicker fusion was determined by decreasing the frequency of signals at a constant speed using a C.F.F. tester (a product of Yagami Co., Ltd.). There was used the average value of the three times values measured by individual eyes of the subjects.

The pattern visual evoked potential was recorded according to the method established by the International Society for Clinical Electrophysiology of Vision to measure one positive peak strength (P100) and the latency (the difference in *µ*V between N75 peak and P100 peak).

### Statistical analysis

The data on before and after administration were analyzed statistically using paired t-test. Also, the data for A and B groups and those for A and C groups were measured in unpaired test. The probability value is below 0.05 that is considered to be significant.

From the above results, no systemic side effect was recognized in B and C groups.

The eyesight of B and C subjects at both the distance of 5 m and 35 cm did not appreciably change before and after administration. Each numerical value of the eye accommodability, the critical flicker fusion and the pattern visual evoked potentials are shown in table below.

Incidentally, in mean ± standard deviation in the table the mark # (p<0.01) was compared with the value before administration, and the mark * (p<0.05) was compared with the value for A group.

**Table 3**

| | Non-VDT Workers | VDT worker | | | |
|---|---|---|---|---|---|
| | A group | B group | | C group | |
| | (n=13 eye) | (n=13 eye) | | (n=13 eye) | |
| | Not Administrated | Before Administration of astaxanthin | After Administration of astaxanthin | Before Administration of placebo | After Administration of placebo |
| Accommodability (D) | 3.7±1.5 | 2.3±1.4 * | 2.8±1.6 # | 22±1.0 * | 2.3±1.1 |
| CFF (Hz) | 45.0±4.2 | 39.9±5.3 | 38.4±4.8 | 39.9±5.5 * | 38.4±3.9 |
| PVEP-P100 (*µ*V) Amplitude | 6.5±1.8 | 5.8±1.7 | 5.6±1.6 | 5.7±2.3 | 5.5±1.3 |
| PVEP-P100 Latency (msec) | 101.3±6.5 | 102.5±6.9 | 104.8±7.4 | 104.4±5.7 | 105.2±5.7 |

The eye-accommodability in A group was 3.7±1.5 dioptres.

The eye-accommodabilities in B and C groups each before administration were 2.3 ± 1.4 dioptres, 2.2 ± 1.0 dioptres, respectively and significantly (p<0.05) lower than in A group.

The eye-accommodability in B group after administration was 2.8±1.6 dioptres, and thus became significantly (p<0.01) greater than before administration.

The eye-accommodability in C group after administration of placebo was 2.3±1.1 dioptres and thus did not (appreciably) change.

The P100 strength in PVEP in A group was 6.5±1.8 µV. The P100 strengths in B and C groups each before administration were 5.8 ± 1.7 *µ*V, 5.7 ± 2.3 *µ*V, respectively each being substantially same with that in A group.

There was no significant difference in the P100 strength between A and B groups. The strengths in B and C groups after administration were respectively 5.6±1.6 *µ*V, 5.5±1.3 *µ*V*,* each being substantially same with that before administration.

The 100 latency in PVEP in A group was 101.3±6.5 msec. The latencies in B and C groups each before administration were respectively 102.5 ± 6.9 msec, 104.4 ± 5.7 msec, each being substantially same with that in A group. There was no significant difference in the latency between B and C groups. The latencies in B and C groups each after administration were respectively 104±7.4 msec, 105.2±5.7 msec, each being same with that in A group.

The eye accommodability may be varied depending on the age. Herein, the age among the three groups was matched one another. Also, diabetes mellitus is a dangerous factor which causes the decrease in the eye-accommodability. And therefore, a diabetes mellitus patient was excluded from this test.

The results of this test show that the eye-accommodability of the VDT workers may be improved by the administration of astaxanthin.

It is reported by Murata et al that in the VDT workers the near point increases and the eye-accommodability decreases (Murata K; Araki S; Kawakami N; Saito Y, Hino E : Central nervous system effects and visual fatigue in VDT workers. Int. Arch Occup Environ Health 1991, 63(2), p109-113) Murata K; Araki S; Yokoyama K; Yamashita K; Okamatsu T; Sakou S : Accumulation of VDT work-related visual fatigue assessed by visual evoked potential, near point distance and critical flicker fusion.Ind. Health 1996, 34(2), 61-69). The authors suggest that the chronic stress caused by use of the VDT induces the hypofunction of cilialy body and decreases the eye-accommodability.

It is reported that in the VDT workers their critical flicker fusion is lowered, their amplitude is decreased and their latency of P100 in PVEP is prolonged.

In this test, a slight critical flicker fusion was seen in the VDT workers. However, the critical flicker fusion was significantly different between before and after the test. In addition, no appreciable decrease in the P100 strength in PVEP was seen in the VDT workers.

In this test, the administration of astaxanthin does not cause any effect toward the critical flicker fusion and the pattern visual evoked potential each derived from nervous system since there is no significant difference in these items between before and after the administration of astaxanthin. On the other hand, the eye-accommodability may be significantly improved by the administration of astaxanthin. This suggests that astaxanthin acts on the cilialy body of eye. The cilialy body does an important action to focus on the object by changing the thickness of lens. It exerts such faction that it stretches in order to make lens thick in near vision while it loosen in far vision.

### Preparation Example 1 (Tablet)

The ingredients shown below were uniformly mixed together in the following composition ratio (wt. %) to make tablets, each 180 mg weight.

| | |
|---|---|
| Astaxanthin | 5 % |
| Lactose | 75 % |
| Ground magnesium oxide | 20 % |

### Preparation Example 2 (Capsule)

*Haematococcus* extracted oil (containing 10 wt. % of astaxanthin) was filled in a soft capsule film consisting of the following components according to a usual method to make soft capsules, each 100 mg weight.

| | |
|---|---|
| Gelatin | 70 % |
| Glycerin | 23% |
| Propyl *p*-hydroxybenzoate | 0.5 % |
| Water | p.q |
| Total | 100% |

### Preparation Example 3 (Capsule)

The above-described *Haematococcus* extracted oil and blueberry extract were filled in the above-described soft capsule film in 1:1 weight ratio according to a usual method to make soft capsules, each 100 mg weight.

### Preparation Example 4 (Drink)

The ingredients shown below were compounded together and water was added thereto according to a usual method to prepare a drink.

| | |
|---|---|
| Astaxanthin | 5 g |
| Liquid sugar | 4 kg |
| Sodium DL-tartrate | 1 g |
| Citric acid | 50 g |
| Vitamin C | 50 g |
| Vitamin E | 150 g |
| Cyclodextrin | 25 g |
| Potassium chloride | 5 g |
| Magnesium sulfate | 2 g |

### Preparation Example 5 (Nutrient and tonic)

The ingredients shown below were compounded together and water was added thereto according to a usual method to prepare a solution.

| | |
|---|---|
| Astaxanthin ethyl ester | 5 g |
| Liquid sugar | 4 kg |
| Sodium DL-tartrate | 1 g |
| Citric acid | 50 g |
| Vitamin B₁ | 10 g |
| Vitamin B₂ | 10 g |
| Vitamin B₆ | 10 g |
| Vitamin B₁₂ | 10 g |
| Vitamin C | 50 g |
| Vitamin E | 150 g |
| Folic acid | 5g |
| Nicotinic acid | 10 g |
| Cyclodextrin | 25 g |
| Potassium chloride | 5 g |
| Magnesium sulfate | 2 g |

### Industrial Applicability

By the present invention there could be provided the use of astaxanthin and/or its esters for the preparation of a medicament for improving failure of the eye-accommodation, and for the preparation of a food and a drink having an improving effect against failure of the -accommodation. As astaxanthin and/or its esters improve the human eye-accommodability, the medicament is useful as a preventive and/or therapeutic agent for the condition where the failure of eye-accommodability occur, such as the presbyopia which shows difficulty in near point accommodation caused by poor accommodability due to aged-deterioration, the weary eye of a person who works for VDT operation, or patients with such morbid abnormalities as weakness of accommodation, hypocyclosis, dullness of accommodation, accommodation paralysis, tonic accommodation, accommodation spasm, etc.

## Claims

1. Use of astaxanthin and/or an ester thereof for the preparation of a medicament for treating a failure of accommodation.

2. The use of claim 1, wherein the astaxanthin and/or an ester thereof is administered orally in a daily amount of 0.1 mg-10 g as free astaxanthin.

3. The use of claim 1, wherein the astaxanthin and/or an ester thereof is administered orally preventively in a daily amount of 0.1-100 mg as free astaxanthin.

4. The use of claim 1, wherein the astaxanthin and/or an ester thereof is administered parenterally in a daily amount of 0.01 mg-1 g as free astaxanthin.

5. The use of claim 1, wherein the astaxanthin and/or an ester thereof is administered parenterally preventively in a daily amount of 0.01-10 mg as free astaxanthin.

6. The use of claim 1, wherein the failure of accommodation is caused by weary eyes of a person who works for VDT operation.

7. The use of claim 1, wherein the failure of accommodation is caused by presbyopia.

8. The use of claim 1, wherein the astaxanthin and/or an ester thereof is administered orally in a food or a drink.

9. Use of astaxanthin and/or an ester thereof for the preparation of a medicament for preventing presbyopia in a subject caused by difficulty in near point accommodation due to age-deterioration or weary eyes of a person who works for VDT operation.

10. The use of claim 9, wherein the astaxanthin and/or an ester thereof is administered orally preventively in a daily amount of 0.1-100 mg as free astaxanthin.

11. The use of claim 9, wherein the astaxanthin and/or an ester thereof is administered parenterally preventively in a daily amount of 0.01-10 mg as free astaxanthin.

## Patentansprüche

1. Verwendung von Astaxanthin und/oder eines Esters davon für die Herstellung eines Medikaments zur Behandlung einer Akkommodationsstörung.

2. Verwendung nach Anspruch 1, wobei das Astaxanthin und/oder ein Ester davon oral in einer täglichen Menge von 0,1 mg - 10 g als freies Astaxanthin verabreicht werden/wird.

3. Verwendung nach Anspruch 1, wobei das Astaxanthin und/oder ein Ester davon oral präventiv in einer täglichen Menge von 0,1-100 mg als freies Astaxanthin verabreicht werden/wird.

4. Verwendung nach Anspruch 1, wobei das Astaxanthin und/oder ein Ester davon parenteral in einer täglichen Menge von 0,01 mg - 1 g als freies Astaxanthin verabreicht werden/wird.

5. Verwendung nach Anspruch 1, wobei das Astaxanthin und/oder ein Ester davon parenteral präventiv in einer täglichen Menge von 0,01-10 mg als freies Astaxanthin verabreicht werden/wird.

6. Verwendung nach Anspruch 1, wobei die Akkommodationsstörung durch ermüdete Augen einer Person, die eine VDT-Tätigkeit ausübt, verursacht wird.

7. Verwendung nach Anspruch 1, wobei die Akkommodationsstörung durch Presbyopie verursacht ist.

8. Verwendung nach Anspruch 1, wobei das Astaxanthin und/oder ein Ester davon oral in einem Nahrungsmittel oder einem Getränk verabreicht werden/wird.

9. Verwendung von Astaxanthin und/oder einem Ester davon für die Herstellung eines Medikaments zur Prävention von Presbyopie bei einer Person, die durch Schwierigkeiten bei der Nahpunktakkommodation durch Altersverschlechterung oder ermüdete Augen einer Person, die eine VDT-Tätigkeit ausübt, verursacht wird.

10. Verwendung nach Anspruch 9, wobei das Astaxanthin und/oder ein Ester davon oral präventiv in einer täglichen Menge von 0,1-100 mg als freies Astaxanthin verabreicht werden/wird.

11. Verwendung nach Anspruch 9, wobei das Astaxanthin und/oder ein Ester davon parenteral präventiv in einer täglichen Menge von 0,01-10 mg als freies Astaxanthin verabreicht werden/wird.

## Revendications

1. Utilisation d'astaxanthine et/ou d'un ester de celle-ci pour la préparation d'un médicament destiné au traitement d'un échec d'accommodation.

2. Utilisation selon la revendication 1, dans laquelle l'astaxanthine et/ou un ester de celle-ci est administré par voie orale en une quantité quotidienne de 0,1 mg à 10g sous la forme d'astaxanthine libre.

3. Utilisation selon la revendication 1, dans laquelle l'astaxanthine et/ou un ester de celle-ci est administré par voie orale de manière préventive en une quantité quotidienne de 0,1 à 100 mg sous la forme d'astaxanthine libre.

4. Utilisation selon la revendication 1, dans laquelle l'astaxanthine et/ou un ester de celle-ci est administré par voie parentérale en une quantité quotidienne de 0,01 mg à 1 g sous la forme d'astaxanthine libre.

5. Utilisation selon la revendication 1, dans laquelle l'astaxanthine et/ou un ester de celle-ci est administré par voie parentérale de manière préventive en une quantité quotidienne de 0,01 à 10 mg sous la forme d'astaxanthine libre.

6. Utilisation selon la revendication 1, dans laquelle l'échec d'accommodation est provoqué par les yeux fatigués d'une personne qui travaille sur un terminal vidéo (VDT).

7. Utilisation selon la revendication 1, dans laquelle l'échec d'accommodation est provoqué par la presbytie.

8. Utilisation selon la revendication 1, dans laquelle l'astaxanthine et/ou un ester de celle-ci est administré par voie orale dans un aliment ou une boisson.

9. Utilisation d'astaxanthine et/ou un ester de celle-ci pour la préparation d'un médicament destiné à prévenir la presbytie chez un sujet, provoquée par la difficulté d'accommodation au punctum proximum due aux yeux fatigués ou détériorés par la vieillesse d'une personne qui travaille sur un terminal vidéo (VDT).

10. Utilisation selon la revendication 9, dans laquelle l'astaxanthine et/ou un ester de celle-ci est administré par voie orale de manière préventive en une quantité quotidienne de 0,1 à 100 mg sous la forme d'astaxanthine libre.

11. Utilisation selon la revendication 9, dans laquelle l'astaxanthine et/ou un ester de celle-ci est administré par voie parentérale de manière préventive en une quantité quotidienne de 0,01 à 10 mg sous la forme d'astaxanthine libre.
